# EUROPÄISCHE PATENTSCHRIFT

(11) **EP 3 364 897 B1**
(45) Veröffentlichungstag und Bekanntmachung des Hinweises auf die Patenterteilung: **12.08.2020**
(21) Anmeldenummer: 16804686.0
(22) Anmeldetag: 12.10.2016
(51) Int. Cl.: A61B 17/72, A61B 17/86, A61B 17/88, A61F 2/46, A61B 17/68, A61B 17/00

(54) **STERILISIERBARES CHIRURGISCHES EINMALINSTRUMENT FÜR DIE KNOCHENFUSIONSCHIRURGIE**
STERILIZABLE DISPOSABLE SURGICAL INSTRUMENT FOR BONE FUSION SURGERY
INSTRUMENT CHIRURGICAL STÉRILISABLE À USAGE UNIQUE POUR LA CHIRURGIE DE FUSION OSSEUSE

(30) Priorität: 19.10.2015 DE 102015013613
(43) Veröffentlichungstag der Anmeldung: 29.08.2018
(73) Patentinhaber: Merete Holding GmbH, 10719 Berlin (DE)
(72) Erfinder: ANAPLIOTIS, Emmanuel, 14195 Berlin (DE)
(74) Vertreter: Bittner, Thomas L.
(86) Internationale Anmeldenummer: PCT/DE2016/000371
(87) Internationale Veröffentlichungsnummer: WO 2017/067532

(56) Entgegenhaltungen:
- US-A1- 2010 131 072
- US-A1- 2013 123 862
- Merete: "MetaToe (TM) ReadyToUse Kit", , 30. April 2016 (2016-04-30), XP055341424, Berlin Gefunden im Internet: URL:http://www.merete-medical.com/de/image s/stories/Publikationen/flyer_metatoe_rtu_ screen.pdf [gefunden am 2017-02-02]
- Merete ET AL: "merete &friends Sterile Winkelstabilität und innovative Osteotomietechnik", , 31. Januar 2016 (2016-01-31), XP055341494, Gefunden im Internet: URL:http://www.merete-medical.com/de/image s/stories/Newsletter/merete_and_friends_rz _screen.pdf [gefunden am 2017-02-02]

## Beschreibung

Die Erfindung betrifft ein sterilisierbares chirurgisches Einmalinstrument sowie ein steril verpacktes Instrumentenset für die Knochenfusionschirurgie, insbesondere für die Interphalangeal-Arthrodese zur Korrektur eines Hammerzehs, das ein Implantat für die Knochenfusion sowie eine Halterungsgriff für das Knochenfusionsimplantat umfasst.

Die Ausbildung eines Hammerzehs ist eine der häufigsten Fehlstellungen der Fußzehen, die sich durch eine Überstreckung des Grundgelenks bei gleichzeitiger Beugung des Endgelenkes zeigt. Hierdurch ist der Zeh ähnlich einem Hammer nach unten gebogen, was zu schmerzhaften Veränderungen wie Hühneraugen oder weiterreichend sogar zu entzündlichen Zustände in den Zehengelenken führt.

Zur Korrektur des Hammerzehs wird das gekrümmte Gelenk geöffnet und anschließend ein oder beide Gelenkköpfchen entfernt. Die Begradigung und Versteifung des Gelenkes erfolgt in der Regel mittels eines Drahtes oder über ein Knochenfusionsimplantat, mit welchem die Gelenkflächen miteinander verbunden werden.

Eine Operationstechnik sieht vor, dass zum Einbringen des Knochenfusionsimplantats in die proximale Gelenkfläche eine Bohrung gesetzt wird, in die der proximale Implantatabschnitt eines Knochenfusionsimplantates eingeschraubt wird. Zum Zusammenbringen der beiden Gelenkflächen wird in die distale Gelenkfläche eine zweite Bohrung gesetzt, die dann den distalen Implantatabschnitt des Knochenfusionsimplantates aufnimmt, wodurch die Gelenkflächen zusammengedrückt werden.

Aus dem Stand der Technik sind Instrumente zum Einbringen von Knochenfusionsimplantaten in einen Knochen bekannt.

So offenbart die US 2014/0277186 A1 ein chirurgisches Instrument für die Interphalangeal-Arthrodese, mit einem Wechselgriff für den Knochenbohrer und den Schraubenschlüssel zum Fixieren des Knochenfusionsimplantats im Knochen.

In der US 2013/0274814 A1 ist ein Knochenfusionsinstrumentarium für die Versteifung eines Hammerzehs beschrieben, welches einen Steckschlüssel zum Einbringen eines zweiteiligen Hammerzeh-Implantates in den Zehenknochen umfasst. Der Steckschlüssel besteht aus einem Griff und einem in den Griff einsteckbaren Werkzeugschaft. Am proximalen Ende des Werkzeugschaftes ist eine Fassung eingerichtet, in der entweder die männliche oder die weibliche Implantatkomponente jeweils mit dem Verbindungsende gelagert gehalten wird.

Ein sterilisierbares chirurgisches Einmalinstrument bestehend aus einem Implantat zur Knochenfusion sowie einer das Implantat vollständig umschließenden Implantathülse ist aus der US 2010/0131072 A1 bekannt.

Nachteilig an den bekannten Instrumentarien für die Implantation von Knochenfusionsimplantaten ist, dass diese als wiederverwendbare Instrumente ausgebildet sind und nach jeder Operation gereinigt, verpackt und erneut sterilisiert werden müssen.

Derartige Instrumente sind in der Herstellung und Anschaffung kostenintensiv, so dass diese in der Regel von Krankenhäusern oder Belegärzten nicht in großen Margen auf Vorrat vorgehalten werden. Dies führt dazu, dass dem Chirurgen für die Durchführung mehrerer gleichartiger Operationen nur eine begrenzte Anzahl an Instrumentarien zur Verfügung steht. Hierdurch beschränkt sich für den Belegarzt die Anzahl der von ihm hintereinander durchführbaren Operationen auf die Anzahl der vorhandenen sterilen Instrumentensets. Anderenfalls erhöht sich für den Belegarzt der Zeitaufwand durch Wartezeiten aufgrund notwendiger Zwischenreinigungen und Sterilisationen der chirurgischen Instrumente.

Aufgabe ist es daher, ein Instrument für das Einsetzen von Knochenfusionsimplantaten, insbesondere ein Instrument für die Interphalangeal-Arthrodese anzugeben, das preisgünstig herstellbar sowie lange steril lagerbar und dadurch auf Vorrat vorhaltbar ist.

Zur Lösung der Aufgabe wird ein sterilisierbares chirurgisches Einmalinstrument bestehend aus einem Implantat zur Knochenfusion sowie einer das Implantat zur Knochenfusion vollständig umschließenden Implantathülse angegeben. Das Implantat zur Knochenfusion weist einen distalen Implantatabschnitt, einen proximalen Implantatabschnitt und einen zwischen dem distalen und dem proximalen Implantatabschnitt befindlichen Kupplungsabschnitt mit einem polygonen Querschnittsprofil auf.

Die Implantathülse besteht aus einer Schutzkappe und einem Griffteil, die miteinander verbindbar ausgestaltet sind. Vorzugsweise kann die Schutzkappe auf das Griffteil aufgesteckt oder aufgeschraubt werden.

In der Schutzkappe ist eine in Längsrichtung verlaufende erste Bohrung eingerichtet, die zur Aufnahme des proximalen Implantatabschnitts des Implantats zur Knochenfusion hergerichtet ist. Der proximale Implantatabschnitt wird durch eine an der distalen Stirnseite der Schutzkappe eingerichteten ersten Öffnung in die erste Bohrung eingeführt und im eingeführten Zustand von der Innenwand der ersten Bohrung flächig umlaufend umschlossen.

Im Griffteil ist eine ebenfalls in Längsrichtung verlaufende Bohrung eingerichtet, die im Weiteren als "zweite Bohrung" bezeichnet wird und zur Aufnahme des distalen Implantatabschnitts des Implantats zur Knochenfusion hergerichtet ist. Der distale Implantatabschnitt wird durch eine an der distalen Stirnseite des Griffteils eingerichteten Öffnung in die zweite Bohrung eingeführt und nach dem Einsetzen in die zweite Bohrung von dessen Innenwand flächig umlaufend umschlossen.

Das erfindungsgemäße Einmalinstrument zeichnet sich dadurch aus, dass in der zweiten Bohrung im Griffteil ein Kupplungsbereich mit einer polygonen Durchgangsöffnung eingerichtet ist, in die der Kupplungsabschnitt des Implantats zur Knochenfusion formschlüssig und kraftschlüssig eingreift.

Damit beschreibt die Erfindung ein Einmalinstrument für die Knochenfusionschirurgie, bei dem ein Knochenfusionsimplantat im Griffteil einer Implantathülse lösbar eingesetzt und vollständig von der Implantathülse umschlossen ist, wenn die Schutzkappe auf das Griffteil aufgesetzt ist. Das Griffteil der Implantathülse ist dafür hergerichtet, das Knochenfusionsimplantat in einen Knochen einzusetzen, vorzugsweise durch Drehen oder Drücken des Griffteils.

Der Begriff "Einmalinstrument" bezeichnet ein chirurgisches Instrument in Form eines Wegwerfmittels, welches nach dessen einmaligem Gebrauch nicht für einen erneuten Einsatz gereinigt, verpackt und sterilisiert wird, sondern direkt entsorgt wird.

Das erfindungsgemäße Implantat zur Knochenfusion, im Kontext auch als Knochenfusionsimplantat bezeichnet, ist vorzugsweise einstückig ausgebildet und besteht im Wesentlichen aus drei ineinander übergehenden Abschnitten, nämlich einem proximalem Bereich, einem distalem Bereich und einem Mittelbereich mit einem Kupplungsabschnitt. Insbesondere handelt es sich bei dem erfindungsgemäßen Implantat zur Knochenfusion um ein Implantat zur Anwendung bei der PIP- oder DIP-Arthrodese, vorzugsweise ein Hammerzeh-Implantat.

Zur Implantation wird das Knochenfusionsimplantat mit dessen proximalem Implantatabschnitt durch Drehen des Griffteils in den Knochen eingedreht und/oder durch Drücken des Griffs in den Knochen hineingedrückt.

Der proximale Implantatabschnitt ist insbesondere ein proximales Implantatgewinde, das ausgehend von der proximalen Gelenkfläche des zu versteifenden Gelenkes in den Knochen, vorzugsweise in dessen Markkanal, somit intramedullär, eingeschraubt wird.

Darüber hinaus kann der proximale Implantatabschnitt jedes weitere für eine Fixierung in einer Knochenbohrung hergerichtete Profil aufweisen. Derartige Profilformen sind insbesondere Rillen- oder Lamellenprofile oder auch Profile mit hakenförmigen Fortsätzen. Auch kann der proximale Implantatabschnitt einen oder mehrere in Längsrichtung verlaufende Längsschnitte aufweisen.

Der distale Implantatabschnitt ist insbesondere ein distales Implantatgewinde, das zur Einbringung in den von der distalen Gelenkfläche ausgehenden Knochen bzw. dessen Markkanal hergerichtet ist. In der Regel wird dieser Implantatabschnitt in eine präparierte Knochenbohrung hineingedrückt bzw. hineingepresst.

Der distale Implantatabschnitt kann alternativ jedoch auch jedwedes weitere für eine Fixierung in einer Knochenbohrung hergerichtete Profil aufweisen, wie vorzugsweise Rillen- oder Lamellenprofile oder auch Profile mit hakenförmigen Fortsätzen. Auch kann der distale Implantatabschnitt einen oder mehrere in Längsrichtung verlaufende Längsschnitte aufweisen.

Der Kupplungsabschnitt mit einem polygonen Querschnittsprofil liegt zwischen dem proximalen und distalen Implantatabschnitt und bildet im Wesentlichen den Mittelbereich des Implantats. Mit polygonem Querschnittsprofil ist ein Profil bezeichnet, welches vorzugsweise ein gleichmäßiges Polygon, insbesondere vier-, sechs- oder achteckig ausgestaltet ist.

Der in der zweiten Bohrung im Griffteil eingerichtet Kupplungsbereich mit einer polygonen Durchgangsöffnung ist vorzugsweise ein gleichmäßiges Polygon, insbesondere vier-, sechs- oder achteckig ausgestaltet. Der Kupplungsbereich ist vorteilhafterweise im Bereich der stirnseitig befindlichen zweiten Öffnung eingerichtet. Das Querschnittsprofil des Kupplungsabschnitts (Implantat) ist pass- und formschlüssig zur Durchgangsöffnung des Kupplungsbereichs (Griffteil) ausgestaltet. Dabei umgreift die Wandung der Durchgangsöffnung radial umlaufend den Kupplungsabschnitt, so dass der Kupplungsabschnitt vollumfänglich von der Wandung der Durchgangsöffnung im Kupplungsbereich eingefasst ist.

Bezeichnend für den Aufbau des Knochenfusionsimplantats ist, dass dessen Kupplungsabschnitt einen größeren Außendurchmesser aufweist als der distale Implantatabschnitt, so dass der distale Implantatabschnitt durch die Durchgangsöffnung hindurch in die zweite Bohrung im Griffteil eingeschoben werden kann.

Das Implantat zur Knochenfusion wird vor der Sterilisation des Einmalinstrumentes in die Implantathülse eingesetzt. Hierzu wird das Knochenfusionsimplantat mit dessen distalem Implantatabschnitt soweit in die zweite Bohrung im Griffteil der Implantathülse eingeschoben, dass der Kupplungsabschnitt des Knochenfusionsimplantats in den Kupplungsbereich der zweiten Bohrung formschlüssig eingreift. Der proximale Implantatabschnitt steht nach dem Einsetzen des Knochenfusionsimplantats aus dem Griffteil heraus.

Der Kupplungsbereich des Griffteils und der Kupplungsabschnitt des Knochenfusionsimplantats sind in deren Passform derart ausgestaltet, dass diese beim Implantieren des Knochenfusionsimplantats in den Knochen einen Kraftschluss ausbilden, der eine Kraftübertragung vom Griffteil auf das Knochenfusionsimplantat ermöglicht. Die Fixierung des distalen Implantatabschnitts im Griffteil erfolgt ausschließlich aufgrund der Passförmigkeit von Durchgangsöffnung und Kupplungsabschnitt.

Die Bohrungen in der Schutzkappe und im Griffteil der Implantathülse sind derart angeordnet, dass beim Verbinden von Schutzkappe und Griffteil die erste Öffnung der ersten Bohrung an die zweite Öffnung der zweiten Bohrung bündig anschließt, so dass bei geschlossener Implantathülse ein Hohlraum ausgebildet mit geschlossenen Wandung wird, in welchem das Knochenfusionsimplantat eingeschlossen ist.

In einer besonderen Ausgestaltung ist die Länge der zweiten Bohrung im Griffteil kleiner oder gleich der summarischen Länge von Kupplungsabschnitt und distalem Implantatabschnitt des Implantats zur Knochenfusion. Bei dieser Ausführungsform schlägt der Endbereich des distalen Implantatabschnitts an der Bodenwandung der zweiten Bohrung an, so dass zum Einschrauben des Knochenfusionsimplantates in den proximalen Knochen auf das Knochenfusionsimplantat in dessen Längsrichtung Druck in Richtung proximalem Knochen ausgeübt werden kann.

In einer Ausführungsform ist die zweite Bohrung im Griffteil als Sacklochbohrung ausgestaltet, dessen der zweiten Öffnung gegenüberliegendes Ende vorzugsweise zur Umgebung geschlossen ausgebildet ist. In einer dazu alternativen Ausführungsform geht die zweite Bohrung im Griffteil in axialer Richtung in eine lineare Durchgangsbohrung mit einem Innendurchmesser, der kleiner als der kleinste Außendurchmesser des distalen Implantatabschnitts ist, über, wobei die Durchgangsbohrung in einer Öffnung im Boden des Griffteils endet.

Diese Durchgangsbohrung ist insbesondere dafür hergerichtet, darin ein Hilfsinstrument zum Herauslösen des Knochenfusionsimplantats aus der formschlüssigen Kupplung mit dem Griffteil zu führen, um das Knochenfusionsimplantat aus der zweiten Bohrung sanft herausdrücken zu können. Vorteilhafterweise ist das Hilfsinstrument ein aus chirurgischem Metall oder Kunststoff gefertigter Stab, der von außen durch die Durchgangsbohrung in die zweite Bohrung im Griffteil eingeführt wird.

In einer vorteilhaften Ausgestaltung besteht das Implantat zur Knochenfusion aus einem bioresorbierbaren bzw. biologisch abbaubaren Material, insbesondere PLGA, aus einem biokompatiblen Kunststoff, vorzugsweise PEEK, oder aus Titan.

Griffteil und Schutzkappe der Implantathülse sind vorteilhafterweise aus einem Kunststoff für Medizinprodukte hergestellt.

Für eine schnelle und einfache Erkennung, welcher Abschnitt der Implantathülse das Griffteil und welcher Abschnitt die Schutzkappe ist, können das Griffteil und die Schutzkappe der Implantathülse optisch und/oder haptisch voneinander unterscheidbar ausgestaltet sein. Bei einer Ausgestaltung mittel optisch unterscheidbarer Mittel können Schutzkappe und Griffteil unterschiedlich eingefärbt sein. Bei einer Unterscheidbarkeit mittels haptischer Mittel können die Schutzkappe und das Griffteil voneinander abweichende Oberflächenstrukturen aufweisen.

In einer bevorzugten Ausgestaltungsform weist das Griffteil zwischen dessen proximaler Stirnseite, auf der die zweite Öffnung der zweiten Bohrung eingerichtet ist, und dessen distalem Ende eine maximale Länge von 30 mm - 40 mm auf. Der maximale Außendurchmesser liegt zwischen 5 mm - 30 mm, vorzugsweise zwischen 7 mm - 15 mm. Durch diese miniaturisierte Ausgestaltungsform, bei der das Griffteil aufgrund dessen geringer Länge nur zwischen den Fingern des Chirurgen führbar ist, werden bei der Verwendung des erfindungsgemäßen Einmalinstrumentes wesentlich geringere Hebel- und Torsionskräfte auf das zu implantierende Knochenfusionsimplantat ausgeübt als bei herkömmlichen längeren chirurgischen Schraubinstrumenten, wodurch die Gefahr von Implantatbrüchen aufgrund der mechanischer Einwirkung durch den Chirurgen deutlich verringert wird. Dies ist insbesondere bei der Implantation von Knochenfusionsimplantaten aus resorbierbaren oder biologisch abbaubaren Materialien, die spröder und brüchiger als nicht abbaubare Kunststoffe oder Metalle sein können, von wesentlichem Vorteil.

Des Weiteren wird beschrieben, ein steril verpacktes Instrumentenset zur Einmalverwendung enthaltend ein chirurgisches Einmalinstrument nach einem der vorgenannten Ausführungsformen, bei dem die Sterilverpackung mindestens aus einem Sterilbarrieresystem und einer Schutzverpackung besteht. Derartige Sterilverpackungen ermöglichen eine sterile Vorratslagerung von bis zu mehreren Jahren.

Vorzugsweise umfasst das steril verpackte Instrumentenset zusätzlich einen Knochenbohrer für die Präparation der Bohrlöcher im Knochen. Zudem kann das Instrumentenset ein Hilfsinstrument zum Herauslösen des Knochenfusionsimplantats zur Knochenfusion aus der formschlüssigen Kupplung mit dem Griffteil umfassen. Ein derartiges Hilfsinstrument kann beispielsweise ein Stößel, Stab oder ein Schieber sein.

Nachstehend wird die Erfindung anhand eines Ausführungsbeispiels näher erläutert. Die Figuren zeigen:
- Fig. 1:: Implantathülse;
- Fig. 2:: Implantat zur Knochenfusion;
- Fig. 3.1 - 3.3:: Einmalinstrument mit geöffneter Implantathülse und eingesetztem Knochenfusionsimplantat und
- Fig. 4.1 und 4.2:: Längsschnitt durch das Einmalinstrument.

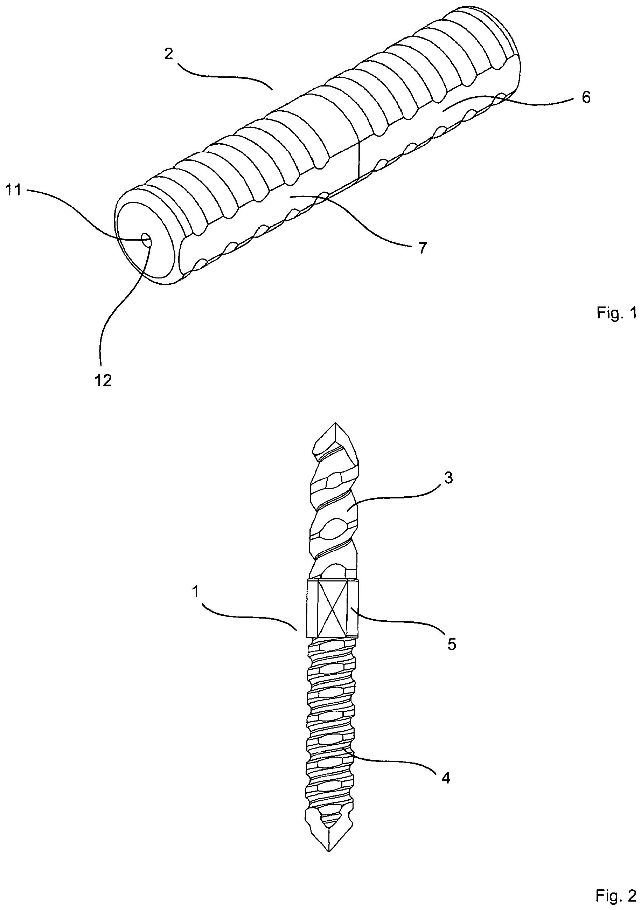

Die Figur 1 zeigt das sterilisierbare chirurgische Einmalinstrument im geschlossenen Zustand. Das Griffteil 7 und die Schutzkappe 6 der Implantathülse 2 sind passförmig aufeinander gesteckt. In Boden des Griffteils 7 ist eine Öffnung 12 eingerichtet, die in die lineare Durchgangbohrung 11 übergeht. In die Öffnung 12 kann ein stabförmiges Hilfsinstrument zum Herausschieben des Knochenfusionsimplantats aus der zweiten Bohrung im Griffteil 7 eingeführt werden.

Die Figur 2 zeigt eine beispielhafte Ausgestaltungform eines Hammerzeh-Implantates 1 mit einem proximalen Gewindeabschnitt 4, einem distalem Gewindeabschnitt 3 sowie einem Kupplungsabschnitt 5 mit einem polygonen Querschnittsprofil.

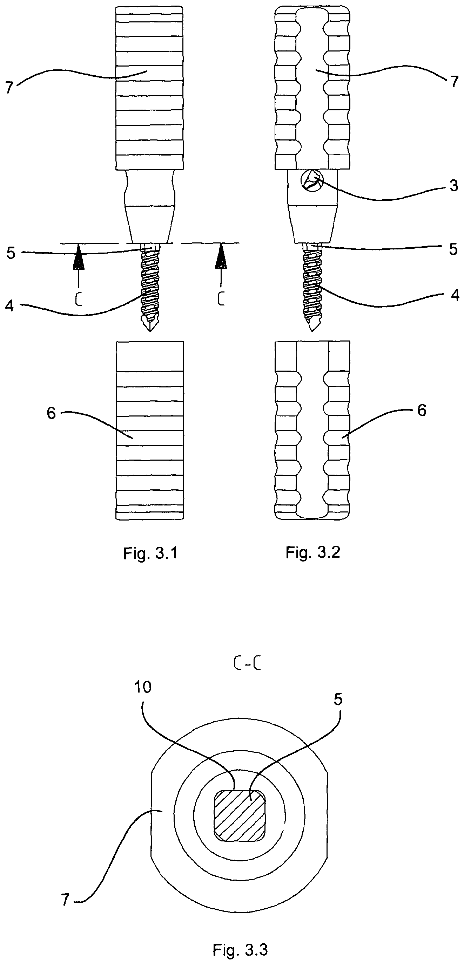

In den Figuren 3.1 bis 3.3 ist das sterilisierbare chirurgische Einmalinstrument im geöffneten Zustand dargestellt. Das Hammerzeh-Implantat 1 ist mit dessen distalem Gewindeabschnitt 3 in das Griffteil 7 eingesetzt, wobei der Kupplungsabschnitt 5 mit seinem im wesentlichen quadratischen Querschnittsprofil in die quadratische Durchgangsöffnung des Kupplungsbereichs 10 formschlüssig eingreift und kraftschlüssig mit diesem zusammenwirkt.

Das Griffteil 7 als auch die Schutzkappe 6 sind mit radial verlaufenden Griffmulden ausgestattet, die eine rutschsichere Rotation zwischen den Fingern erlaubt. In Längsrichtung weisen sowohl das Griffteil 7 als auch die Schutzkappe 6 plane Flächen auf, die einerseits ein Wegrollen des Einmalinstrumentes auf einer glatten Oberfläche verhindern als auch die Kraftausübung des Chirurgen auf das Einmalinstrument z.B. beim Eindrehen des Knochenfusionsimplantats 1 in den proximalen Knochen verbessern.

Der proximale Bereich des Griffteils 7 weist einen geringen Außendurchmesser als der distale Bereich des Griffteils 7 auf und läuft zudem zum proximalen Ende konisch zusammen. Auf diesen Bereich wird die Schutzkappe 6 zum Schließen der Implantathülse 2 aufgesteckt, um den proximalen Implantatabschnitt 4 abzudecken.

Die Schutzkappe 6 ist länger als der aus dem Griffteil 7 herausstehende proximale Gewindeabschnitt 4 des Knochenfusionsimplantats 1 ausgestaltet, so dass die Schutzkappe 6 den proximalen Gewindeabschnitt 4 vollständig umschließt, wenn die Implantathülse 2 durch Aufstecken der Schutzkappe 6 auf das Griffteil 7 geschlossen wird.

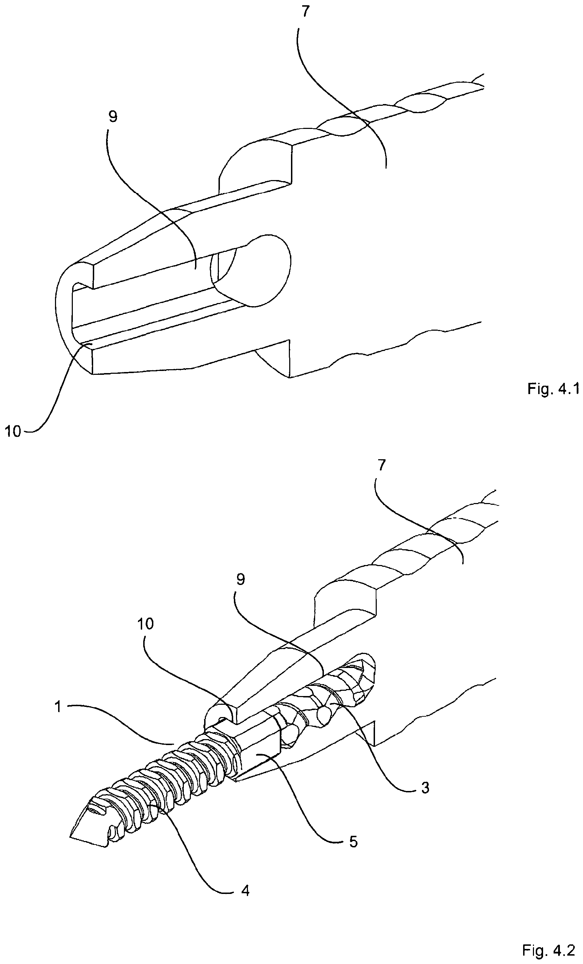

Die Figuren 4.1 und 4.2 stellen das Griffteil 7 des erfindungsgemäßen Einmalinstrumentes in einem Längsschnitt dar. Dargestellt sind die zweite Bohrung 9, die als Sacklochbohrung ausgestaltet ist, sowie der Kupplungsbereich 10 mit einer quadratischen Durchgangsöffnung.

Der distale Gewindeabschnitt 3 des Knochenfusionsimplantats 1 ist in die Sacklochbohrung 9 bis zum Anschlag ans Bohrlochende eingeschoben. Der Kupplungsabschnitt 5 mit einem achteckigen Querschnittsprofil greift im Wesentlichen formschlüssig in die quadratische Durchgangsöffnung des Kupplungsbereiches 10 ein. Der proximale Gewindeabschnitt 4 des Knochenfusionsimplantats 1 steht aus dem Griffteil 7 vollständig hervor.

### Bezugszeichen:

1) Implantat zur Knochenfusion
2) Implantathülse
3) distaler Implantatabschnitt,
4) proximaler Implantatabschnitt
5) Kupplungsabschnitt mit einem polygonen Querschnittsprofil
6) Schutzkappe
7) Griffteil
8) erste Bohrung zur Aufnahme des proximalen Gewindebereichs des Knochenfusionsim plantats
9) zweite Bohrung zur Aufnahme des distalen Gewindebereichs des Knochenfusionsim plantats
10) Kupplungsbereich mit einer polygonen Durchgangsöffnung im Griffteil
11) lineare Durchgangsbohrung im Griffteil
12) Öffnung im Boden des Griffteils

## Patentansprüche

1. Sterilisierbares chirurgisches Einmalinstrument bestehend aus einem Implantat zur Knochenfusion (1) sowie einer das Implantat zur Knochenfusion (1) vollständig umschließenden Implantathülse (2),
wobei das Implantat zur Knochenfusion (1) einen distalen Implantatabschnitt (3), einen proximalen Implantatabschnitt (4) und einen zwischen distalem (3) und proximalen Implantatabschnitt (4) befindlichen Kupplungsabschnitt (5) mit einem polygonen Querschnittsprofil aufweist,
wobei die Implantathülse (2) aus einer Schutzkappe (6) und einem Griffteil (7) besteht, die miteinander verbindbar ausgestaltet sind,
wobei in der Schutzkappe (6) eine in Längsrichtung verlaufende erste Bohrung (8) eingerichtet ist, die zur Aufnahme des proximalen Implantatabschnitts (4) des Implantats zur Knochenfusion (1) hergerichtet ist, wobei im Griffteil (7) eine in Längsrichtung verlaufende zweite Bohrung (9) eingerichtet ist, die zur Aufnahme des distalen Implantatabschnitts (3) des Implantats zur Knochenfusion (1) hergerichtet ist,
**dadurch gekennzeichnet, dass**
in der zweiten Bohrung (9) im Griffteil (7) ein Kupplungsbereich (10) mit einer Durchgangsöffnung mit einem polygonen Querschnitt eingerichtet ist, in die der Kupplungsabschnitt (5) des Implantats zur Knochenfusion (1) form- und kraftschlüssig eingreift.

2. Sterilisierbares chirurgisches Einmalinstrument nach Anspruch 1, **dadurch gekennzeichnet, dass**
die zweite Bohrung (9) im Griffteil (7) eine Sacklochbohrung ist.

3. Sterilisierbares chirurgisches Einmalinstrument nach Anspruch 1, **dadurch gekennzeichnet, dass**
die zweite Bohrung (9) im Griffteil (7) in axialer Richtung in eine lineare Durchgangsbohrung (11) mit einem Innendurchmesser, der kleiner als der kleinste Außendurchmesser des distalen Implantatabschnitts ist, übergeht, wobei die Durchgangsbohrung in einer Öffnung im Boden (12) des Griffteils endet.

4. Sterilisierbares chirurgisches Einmalinstrument nach einem der Ansprüche 1 bis 3, **dadurch gekennzeichnet, dass**
das Implantat zur Knochenfusion (1) aus einem bioresorbierbaren Material, insbesondere PLGA, aus einem biokompatiblen Kunststoff, vorzugsweise PEEK, oder aus Titan besteht.

5. Sterilisierbares chirurgisches Einmalinstrument nach einem der Ansprüche 1 bis 4, **dadurch gekennzeichnet, dass**
die Implantathülse (2) vollständig aus einem Kunststoff für Medizinprodukte hergestellt ist.

6. Sterilisierbares chirurgisches Einmalinstrument nach einem der Ansprüche 1 bis 5, **dadurch gekennzeichnet, dass**
das Griffteil (7) und die Schutzkappe (6) der Implantathülse (2) optisch und/oder haptisch voneinander unterscheidbar ausgestaltet sind.

7. Sterilisierbares chirurgisches Einmalinstrument nach einem der Ansprüche 1 bis 6, **dadurch gekennzeichnet, dass**
die Länge der zweiten Bohrung (9) im Griffteil (7) kleiner oder gleich der summarischen Länge von Kupplungsabschnitt (5) und distalem Implantatabschnitt (3) des Implantats zur Knochenfusion (1) ist.

8. Sterilisierbares chirurgisches Einmalinstrument nach einem der Ansprüche 1 bis 7, **dadurch gekennzeichnet, dass**
das Griffteil (7) eine maximale Länge von 30 mm - 40 mm und einen maximalen Außendurchmesser von 5 mm - 30 mm, vorzugsweise von 7 mm - 15 mm aufweist.

9. Sterilisierbares chirurgisches Einmalinstrument nach einem der Ansprüche 1 bis 8, **dadurch gekennzeichnet, dass**
das sterilisierbare chirurgische Einmalinstrument ein Hilfsinstrument zum Herauslösen des Implantats zur Knochenfusion aus der formschlüssigen Kupplung mit dem Griffteil umfasst.

## Claims

1. Sterilizable single-use surgical instrument consisting of an implant for bone fusion (1) and an implant sleeve (2) completely enclosing the implant for bone fusion (1),
wherein the implant for bone fusion (1) has a distal implant portion (3), a proximal implant portion (4) and a coupling portion (5) which has a polygonal cross-sectional profile and is located between the distal (3) and proximal implant portion (4),
wherein the implant sleeve (2) consists of a protective cap (6) and a handle portion (7) which are designed to be connectable to one another,
wherein a first bore (8) running in the longitudinal direction is provided in the protective cap (6) and is arranged to receive the proximal implant portion (4) of the implant for bone fusion (1), wherein a second bore (9) running in the longitudinal direction is provided in the handle portion (7) and is arranged to receive the distal implant portion (3) of the implant for bone fusion (1),
**characterized in that** a coupling region (10) having a through opening with a polygonal cross section is provided in the second bore (9) in the handle portion (7), into which opening the coupling portion (5) of the implant for bone fusion (1) engages in a form-fitting and force-fitting manner.

2. Sterilizable single-use surgical instrument according to claim 1, **characterized in that** the second bore (9) in the handle portion (7) is a blind hole bore.

3. Sterilizable single-use surgical instrument according to claim 1, **characterized in that** the second bore (9) in the handle portion (7) merges in the axial direction into a linear through bore (11) with an inner diameter which is smaller than the smallest outer diameter of the distal implant portion, wherein the through bore ends in an opening in the bottom (12) of the handle portion.

4. Sterilizable single-use surgical instrument according to one of claims 1 to 3, **characterized in that** the implant for bone fusion (1) consists of a bioresorbable material, in particular PLGA, of a biocompatible plastics material, preferably PEEK, or of titanium.

5. Sterilizable single-use surgical instrument according to one of claims 1 to 4, **characterized in that** the implant sleeve (2) is made entirely of a plastics material for medical products.

6. Sterilizable single-use surgical instrument according to one of claims 1 to 5, **characterized in that** the handle portion (7) and the protective cap (6) of the implant sleeve (2) are designed to be optically and/or haptically distinguishable from one another.

7. Sterilizable single-use surgical instrument according to one of claims 1 to 6, **characterized in that** the length of the second bore (9) in the handle portion (7) is less than or equal to the sum of the lengths of the coupling portion (5) and distal implant portion (3) of the implant for bone fusion (1).

8. Sterilizable single-use surgical instrument according to one of claims 1 to 7, **characterized in that** the handle portion (7) has a maximum length of 30 mm - 40 mm and a maximum outer diameter of 5 mm - 30 mm, preferably of 7 mm - 15 mm.

9. Sterilizable single-use surgical instrument according to one of claims 1 to 8, **characterized in that** the sterilizable single-use surgical instrument comprises an auxiliary instrument for releasing the implant for bone fusion from the form-fitting coupling with the handle portion.

## Revendications

1. Instrument chirurgical stérilisable à usage unique constitué d'un implant pour fusion osseuse (1) et d'un manchon d'implant (2) entourant complètement l'implant pour fusion osseuse (1),
l'implant pour fusion osseuse (1) présentant une section d'implant distale (3), une section d'implant proximale (4) et une section de couplage (5) ayant un profil transversal polygonal situé entre les sections d'implant distale (3) et proximale (4),
le manchon d'implant (2) étant constitué d'un capuchon de protection (6) et d'une partie de préhension (7), qui sont conçus de manière à pouvoir être reliés,
un premier alésage (8) s'étendant dans le sens longitudinal étant créé dans le capuchon de protection (6), lequel premier alésage est conçu pour recevoir la section d'implant proximale (4) de l'implant pour fusion osseuse (1), un second alésage (9) s'étendant dans le sens longitudinal étant créé dans la partie de préhension (7), lequel second alésage est conçu pour recevoir la section d'implant distal (3) de l'implant pour fusion osseuse (1),
**caractérisé en ce que**, dans le second alésage (9) situé dans la partie de préhension (7), il est créé une zone de couplage (10) ayant une ouverture de passage présentant une section polygonale, zone de couplage dans laquelle la section de couplage (5) de l'implant pour fusion osseuse (1) s'engage par complémentarité de forme et à force.

2. Instrument chirurgical stérilisable à usage unique selon la revendication 1, **caractérisé en ce que** le second alésage (9) situé dans la partie de préhension (7) est un alésage borgne.

3. Instrument chirurgical stérilisable à usage unique selon la revendication 1, **caractérisé en ce que** le second alésage (9) situé dans la partie de préhension (7) rejoint, dans le sens axial, un alésage de passage linéaire (11) ayant un diamètre intérieur plus petit que le plus petit diamètre extérieur de la section d'implant distal, l'alésage de passage se terminant par une ouverture au fond (12) de la partie de préhension.

4. Instrument chirurgical stérilisable à usage unique selon l'une des revendications 1 à 3, **caractérisé en ce que** l'implant pour fusion osseuse (1) est constitué d'un matériau biorésorbable, en particulier du PLGA, d'une matière plastique biocompatible, de préférence du PEEK, ou de titane.

5. Instrument chirurgical stérilisable à usage unique selon l'une des revendications 1 à 4, **caractérisé en ce que** le manchon d'implant (2) est entièrement réalisé en une matière plastique pour dispositifs médicaux.

6. Instrument chirurgical stérilisable à usage unique selon l'une des revendications 1 à 5, **caractérisé en ce que** la partie de préhension (7) et le capuchon de protection (6) du manchon d'implant (2) sont conçus de façon à pouvoir être différenciés par voie optique et/ou haptique.

7. Instrument chirurgical stérilisable à usage unique selon l'une des revendications 1 à 6, **caractérisé en ce que** la longueur du second alésage (9) situé dans la partie de préhension (7) est inférieure ou égale à la longueur totale de la section de couplage (5) et de la section d'implant distale (3) de l'implant pour fusion osseuse (1).

8. Instrument chirurgical stérilisable à usage unique selon l'une des revendications 1 à 7, **caractérisé en ce que** la partie de préhension (7) a une longueur maximale comprise entre 30 et 40 mm et un diamètre extérieur maximal compris entre 5 et 30 mm, de préférence entre 7 et 15mm.

9. Instrument chirurgical stérilisable à usage unique selon l'une des revendications 1 à 8, **caractérisé en ce que** l'instrument chirurgical stérilisable à usage unique comprend un instrument auxiliaire servant à retirer l'implant pour fusion osseuse du couplage par complémentarité de forme avec la partie de préhension.
